(19) Europäisches Patentamt
European Patent Office
Office européen des brevets

(11) Veröffentlichungsnummer : **0 415 200 B1**

# (12) EUROPÄISCHE PATENTSCHRIFT

(45) Veröffentlichungstag der Patentschrift :
**16.02.94 Patentblatt 94/07**

(51) Int. Cl.$^5$ : **C07C 69/732,** C08F 20/30

(21) Anmeldenummer : **90115747.9**

(22) Anmeldetag : **17.08.90**

## (54) Bisphenolderivate und deren Polymerisate.

(30) Priorität : **30.08.89 DE 3928658**

(43) Veröffentlichungstag der Anmeldung :
**06.03.91 Patentblatt 91/10**

(45) Bekanntmachung des Hinweises auf die Patenterteilung :
**16.02.94 Patentblatt 94/07**

(84) Benannte Vertragsstaaten :
**DE FR GB IT**

(56) Entgegenhaltungen :
**EP-A- 0 182 585**
**DE-A- 3 029 026**

(73) Patentinhaber : **BAYER AG**
**D-51368 Leverkusen (DE)**

(72) Erfinder : **Lütjens, Holger, Dr.**
**Rybniker Strasse 12**
**D-5000 Köln 80 (DE)**
Erfinder : **Westeppe, Uwe, Dr.**
**Vogelskamp 72**
**D-4020 Mettmann (DE)**
Erfinder : **Piejko, Karl-Erwin, Dr.**
**Unterscheider Weg 7a**
**D-5060 Bergisch Gladbach 2 (DE)**
Erfinder : **Lindner, Christian, Dr.**
**Im Wasserblech 11**
**D-5000 Köln 80 (DE)**

EP 0 415 200 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft spezielle Bisphenolderivate und aus ihnen hergestellte Polymerisate. Die diese Bisphenole in polymerisierter Form enthaltenden Polymerisate besitzen besondere Eigenschaften und technische Anwendungsmöglichkeiten.

Gegenstand der Erfindung sind Bisphenolderivate der Formel (I)

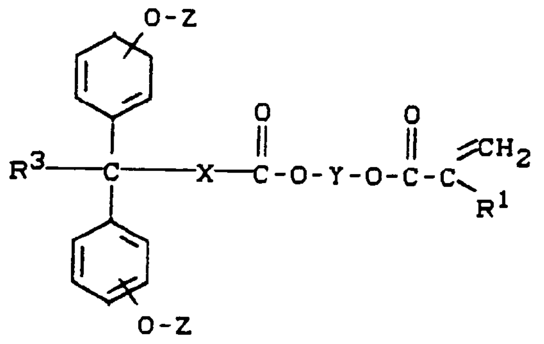

(I)

worin

R¹ Wasserstoff, $C_1$-$C_4$-Alkyl (bevorzugt Methyl),
R² $C_1$-$C_{12}$-Alkyl (bevorzugt Methyl),
R³ $C_1$-$C_{12}$-Alkyl,
X $C_1$-$C_{12}$-Alkylen, bevorzugt $C_1$-$C_8$-Alkylen
Y $C_1$-$C_{12}$-Alkylen, bevorzugt $C_1$-$C_8$-Alkylen
Z H,

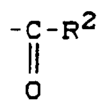

bedeuten.

Ein weiterer Gegenstand der Erfindung sind Polymerisate enthaltend Struktureinheiten der Formel (II)

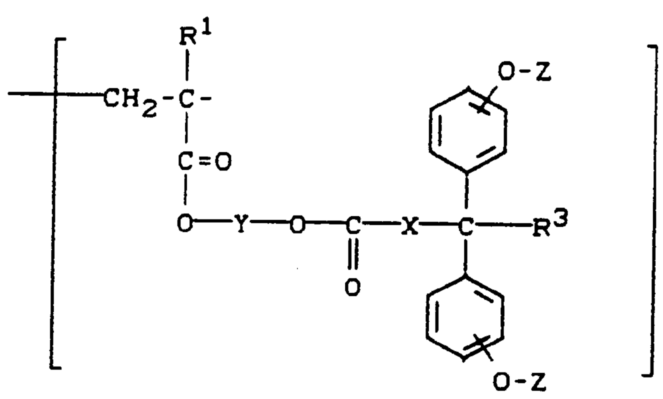

(II)

worin

R¹, R², R³, X, Y und Z die obengenannte Bedeutung haben.

Die erfindungsgemäßen Bisphenolderivate der Formel (I) können durch Umsetzung einer Bis-(4-acetoxyphenyl)carbonsäure oder eines Derivats der Formel (III) mit einem Hydroxyalkylacrylat der Formel (IV) hergestellt werden.

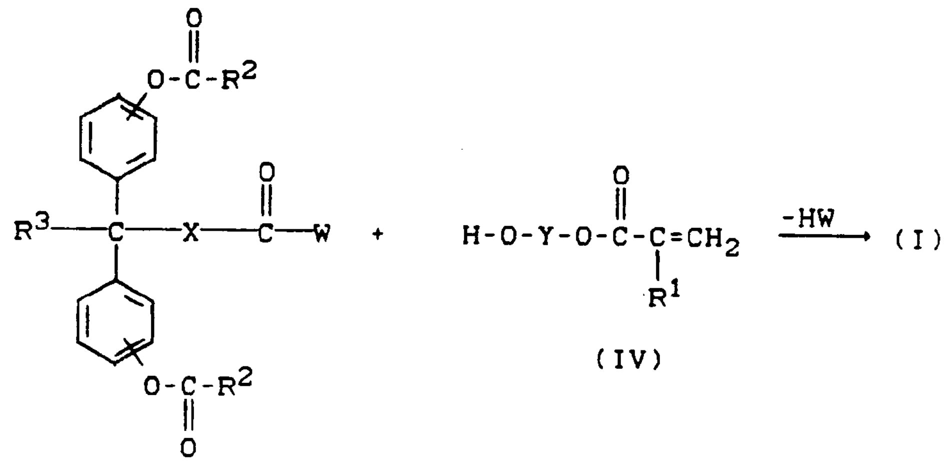


(III) (IV) (I)

wobei

$R^1$ , $R^2$, $R^3$, X, Y die obengenannte Bedeutung haben und

W für OH, $OR^4$, $N(R^4)_2$, Hal (bevorzugt Cl) und $R^4$ für $C_1$-$C_{12}$-Alkyl steht.

Bevorzugte Derivate der Formel (III) sind die Carbonsäurechloride (W = Cl).

Als Ausgangsprodukte geeignete Hydroxyalkylacrylate der Formel (IV) sind z.B.

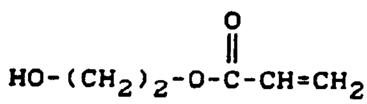

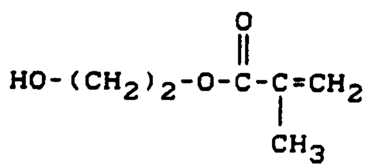

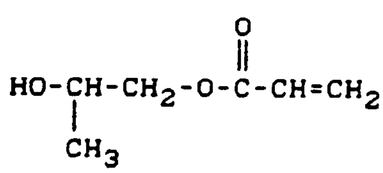

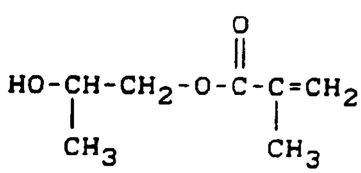

und geeignete Carbonsäurehalogenide der Formel (III) sind z.B.

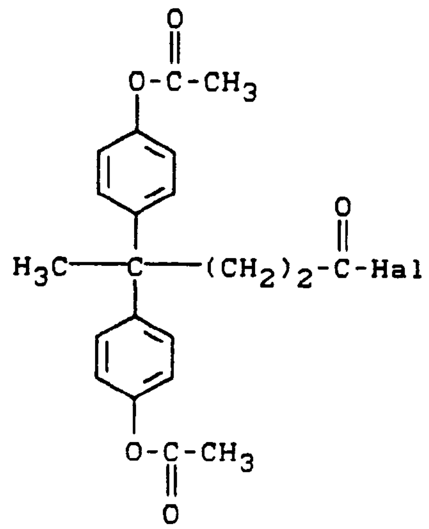

Hal: Cl, Br

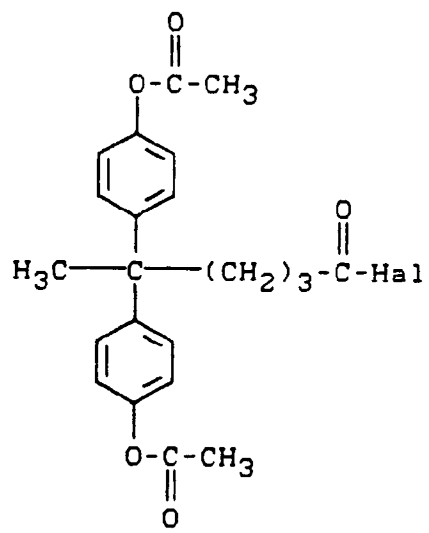

Hal: Cl, Br

Die erfindungsgemäßen Polymerisate mit Struktureinheiten der Formel (II) sind Homopolymerisate der Bisphenolderivate der Formel (I) oder Copolymerisate, die Struktureinheiten der Formel (II) neben anderen Struktureinheiten enthalten. Die Copolymerisate der Erfindung enthalten Struktureinheiten der Formel (II) in Mengen von 1 bis 99 Gew.-%. Die in den Copolymerisaten enthaltene anderen Struktureinheiten leiten sich bevorzugt ab von den folgenden Monomeren:

a) Vinylverbindungen wie Styrol, α-Methylstyrol, Halogenstyrole, Methoxystyrole,
b) Vinylhalogenide wie Vinylchlorid, Vinylfluorid, Vinylidenchlorid,
c) Vinylalkylketone wie Vinylmethylketon,
d) Vinylester organischer Säuren, wie z.B. Vinylacetat, Vinylbutyrat, Vinylpropionat,
e) α,β-ungesättigte Carbonsäuren wie Acrylsäure, Methacrylsäure, Itaconsäure, Maleinsäure,
f) Derivate der Acrylsäure und der Methacrylsäure wie Acrylnitril, Methacrylnitril, Acrylsäureamid, Methacrylsäureamid, N-Methylolacrylamid, N-Methylolmethacrylamid, Dimethylaminopropylamid,
g) $C_1$-$C_{14}$-Alkylacrylate, wie Methyl-, Ethyl-, Butyl-, Octyl-, 2-Ethylhexylacrylat, Chlorethylacrylat Benzylacrylat, Phenylethylacrylat;
$C_1$-$C_8$-Alkylmethacrylate, die im Alkylrest gegebenenfalls durch funktionelle Gruppen wie Hydroxyl-, Epoxid-, Oxetan-, Amin-Gruppen substituiert sein können, beispielsweise Methyl-, Cyclohexylmethacrylat, Glycidylmethacrylat, Hydroxyethyl-, Hydroxypropylmethacrylat, Dimethylaminoethylmethacrylat; Maleinsäureanhydrid,
h) polymerisierbare Olefine und Diene wie Isobutylen, Butadien, Isopropen, Propylen, Chloropren,
i) Vinylalkylether.

Die erfindungsgemäßen Polymerisate können in an sich bekannter Weise durch Polymerisation aus den erfindungsgemäßen Bisphenolderivaten der Formel (I) und gegebenenfalls den obengenannten Comonomeren hergestellt werden. Die Polymerisation wird bevorzugt als Lösungs-, Suspensions- oder Emulsionspolymerisation und bevorzugt in Gegenwart radikalischer Initiatoren durchgeführt. Geeignete radikalische Initiatoren sind beispielsweise azogruppenhaltige Verbindungen, wie Azoisobutyrodinitril, 4,4-Azobis-(4-cyanovaleriansäure), organische Peroxide wie Benzoylperoxid, tert.-Butylhydroperoxid, Dibenzoylperoxid und anorganische Peroxidsalze wie Kaliumperoxodisulfat, Ammoniumperoxodisulfat.

Wird die Polymerisation in Lösung vorgenommen, können Lösungsmittel verwendet werden, in denen nur die Monomeren löslich sind oder solche, in denen die Monomeren und die Polymerisate löslich sind. Geeignete organische Lösungsmittel sind beispielsweise Butanol, Methylethylketon, Ethylbenzol.

Wird die Polymerisation in (wäßriger) Emulsion durchgeführt, werden die Monomeren zweckmäßig mit Hilfe von Emulgatoren z.B. anionaktiven, kationaktiven oder nichtionogenen Emulgatoren emulgiert, beispielsweise mit Natrium-, Kalium-, Ammonium-Salzen von Fettsäuren, Natriumlaurylsulfat, Natriumsalz von $C_{14}$-$C_{18}$-Alkylsulfonsäuren, Oleyl- oder Octadecylalkohol.

Polymerisiert wird vorteilhaft bei erhöhter Temperatur z.B. bei +30 bis +90°C, insbesondere bei +60 bis +85°C.

Zur Polymerisation sind in den Bisphenolderivaten der Formel (I) die Reste Z bevorzugt gleich

$$-\overset{\displaystyle O}{\overset{\|}{C}}-R^2 \quad ,$$

wobei diese Reste auch während der Polymerisation erhalten bleiben; freie Phenolgruppen können die Polymerisation inhibieren. Man sollte auch bei der Emulsionspolymerisation stark saures (pH <1) und stark alkalisches (pH >12) Milieu bei höheren Temperaturen vermeiden. Bevorzugt wird die Polymerisation in schwach saurem oder neutralem Milieu durchgeführt.

Das Molekulargewicht der erfindungsgemäßen Polymerisate kann in bekannter Weise durch Polymerisationstemperatur, Monomeren-Konzentration, Katalysatormenge und durch Molekulargewichtsregler eingestellt werden. Bevorzugte Molekulargewichtsregler sind Organoschwefelverbindungen, z.B. Mercaptane oder Disulfide, insbesondere langkettige Mercaptane wie n- und tert.-Dodecylmercaptane. Sie werden normalerweise im Monomer gelöst. Die Molekulargewichte $\overline{M}w$ (Gewichtsmittel) der erfindungsgemäßen Polymerisate (durch Lichtstreuung oder Sedimentation ermittelt) sind 1000 bis 5 000 000 g/mol.

Durch Basen können die Reste

$$Z = \quad -\overset{\displaystyle O}{\overset{\|}{C}}-R^2$$

in den erfindungsgemäßen Polymerisaten hydrolysiert werden, so daß Struktureinheiten der Formel (II) mit Z = H entstehen.

Zur Hydrolyse kann man beispielsweise zunächst ohne Isolierung der Polymerisate den Polymerisationsansatz (z.B. die Emulsion) nach Beendigung der Polymerisation mit einer Base versetzen und dann die Emulsion längere Zeit auf 30°C bis 95°C, vorzugsweise 50 bis 90°C z.B. 3 Stunden bis 3 Tage, vorzugsweise 12 bis 24 Stunden lang erhitzen und danach die Polymerisate durch Koagulation oder Fällung isolieren. Geeignete Basen sind Alkalimetallhydroxide, beispielsweise Kaliumhydroxid, Natriumhydroxid, Ammoniumhydroxid, quartäre Ammonium-Basen, beispielsweise Tetramethylammoniumhydroxid, Benzyltrimethylammoniumhydroxid und wasserlösliche Amine, beispielsweise Methylamin, Ethylamin, Dimethylamin, Diethylamin, Benzylmethylamin.

Die erfindungsgemäßen Polymerisate können nach Umsetzung der phenolischen Gruppen mit Epichlorhydrin als Epoxidharze verwendet werden. Sie eignen sich ebenfalls als Epoxidharzvernetzer und können außerdem bei der Herstellung von Photoresists verwendet werden. Sie wirken auch als migrationsfeste polymere Stabilisatoren (Antioxidantien) aufgrund ihrer Phenolgruppen.

Beispiele

Beispiel 1.1:

Zu 286 g (1 Mol) 4,4-Bis-(4-hydroxyphenyl)-valeriansäure (95 %, Fa. Aldrich-Chemie) werden unter Rühren bei Raumtemperatur 224 g (2,2 Mol) Acetanhydrid gegeben und die Reaktionsmischung mit 0,5 ml konzentrierter Schwefelsäure versetzt. Nach 2-stündigem Rühren bei 100°C wird die Reaktionsmischung auf Eis gegeben und mit Methylenchlorid extrahiert. Die organischen Extrakte werden mit wäßriger Natriumhydrogencarbonatlösung und Wasser gewaschen und getrocknet und das Lösungsmittel abdestilliert. Das verbleibende, feste Rohprodukt wird mit kaltem Toluol und Hexan gewaschen.
Ausbeute: 295 g (80 %)

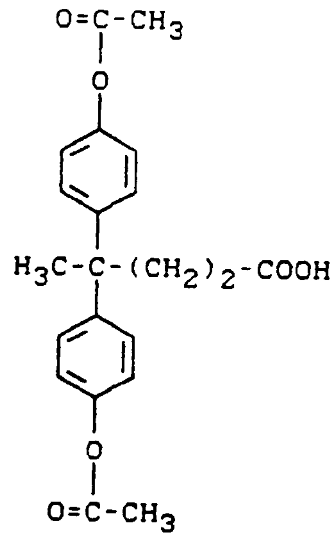

Schmelzpunkt: 140 bis 142°C.

Beispiel 1.2:

93 g (0,25 Mol) gemäß Beispiel 1.1 werden mit 45 g (0,38 Mol) Thionylchlorid und 1 Tropfen Dimethylformamid versetzt. Die Reaktionsmischung wird langsam unter Rühren auf 90°C erhitzt und 1 Stunde bei dieser Temperatur gehalten. Das überschüssige Thionylchlorid wird dann im Vakuum abdestilliert und der verbleibende, feste Rückstand mit kaltem Toluol und Hexan gewaschen.
Ausbeute: 84 g (85 %)

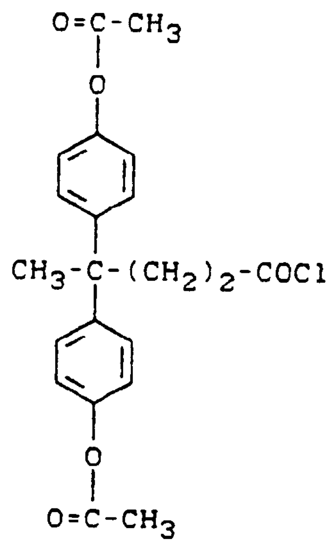

Schmelzpunkt: 113°C.

Beispiel 1.3 (erfindungsgemäß):

Zu einer Lösung von 65 g (0,5 Mol) 2-Hydroxyethylmethacrylat und 62 g (0,6 Mol) Triethylamin in 300 ml Methylenchlorid wird bei 0°C unter Rühren langsam eine Lösung von 195 g (0,5 Mol) gemäß Beispiel 1.2 in 400 ml Methylenchlorid zugetropft und 12 Stunden bei Raumtemperatur weitergerührt. Nach Abfiltrieren des unlöslichen wird das Methylenchlorid abdestilliert. Es verbleibt ein klares, viskoses, leicht gelblich gefärbtes Öl. Ausbeute: 225 g (90 %).

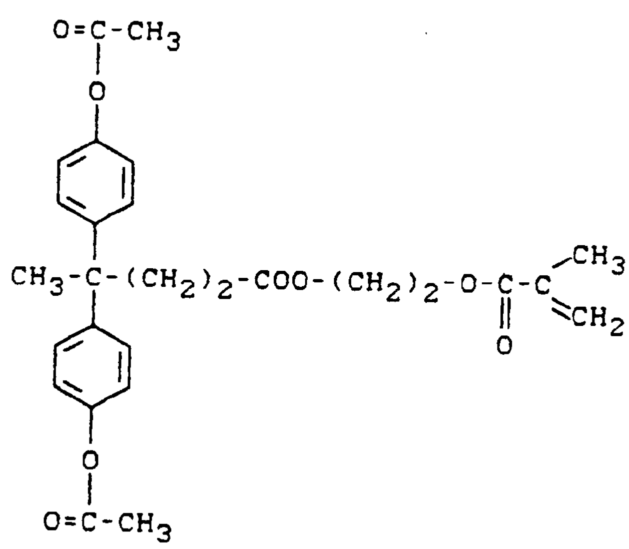

Das Produkt wird wie folgt analysiert:

IR (Film): $\nu$ = 3000, 1760, 1740, 1640, 1610, 1510, 1370, 1320, 1300, 1200, 1170, 1020, 920, 850 $cm^{-1}$.

$^1H$-NMR ($CDCl_3$, TMS): $\delta$ = 7,20 (m; 4H), 7,00 (m; 4H), 6,13 (m; 1H), 5,60 (m; 1H), 4,30 (m; 4H), 2,42 (m; 2H), 2,30 (s; 6H), 2,15 (m; 2H), 1,95 (s; 3H), 1,60 (s; 3H) ppm.

m = Multiplett, s = Singulett
TMS: Tetramethylsilan
MG: theoretisch: 482 g/mol
MG: dampfdruckosmometrisch: 479 g/mol

Beispiel 1.4:

Das Beispiel 1.3 wird wiederholt, indem man anstelle des 2-Hydroxyethylmethacrylats 2-Hydroxyethylacrylat einsetzte.
Ausbeute: 217 g (87 %),

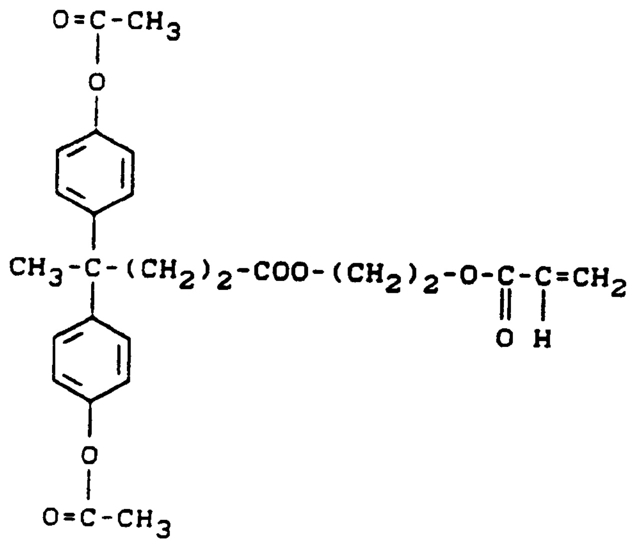

viskoses Öl.

IR (Film): $\nu$ = 3000, 1760, 1730, 1640, 1610, 1500, 1410, 1370, 1200, 1040, 1020, 910, 850 $cm^{-1}$.
MG theoretisch: 468 g/mol
MG dampfdruckosmometrisch: 462 g/mol

Beispiel 2 (erfindungsgemäß):

Die Lösung von 13 Gew.-Teilen der nachfolgenden Monomermischungen in 52 Gew.-Teile Ethylbenzol wird mit 0,26 Gew.-Teilen Azoisobutyronitril versetzt und unter Rühren 15 Stunden unter Stickstoff auf 65°C erhitzt. Die Lösung wird dann in Methanol eingerührt, das ausgefallene Polymerisat abfiltriert und getrocknet.

| Beispiel | Monomer gemäß Beispiel 1.3 Gew.-Teile | Styrol Gew.-Teile | MMA Gew.-Teile | n-BA Gew.-Teile |
|---|---|---|---|---|
| 2.1 | 13 | - | - | - |
| 2.2 | 10 | 3 | - | - |
| 2.3 | 10 | - | 3 | - |
| 2.4 | 10 | - | - | 3 |

MMA: Methylmethacrylat; n-BA: n-Butylacrylat

Ausbeute:

Beispiel 2.1: 10,8 Gew.-Teile (83 %)
Beispiel 2.2: 12,0 Gew.-Teile (92 %)

Beispiel 2.3: 12,3 Gew.-Teile (95 %)
Beispiel 2.4: 12,4 Gew.-Teile (93 %)

Das Polymerisat gemäß Beispiel 2.1 ergibt folgende Analyse:

| berechnet | gefunden |
|---|---|
| C = 67,2 % | C = 67,8 % |
| H = 6,3 % | H = 6,4 % |

Das Polymerisat enthält Struktureinheiten

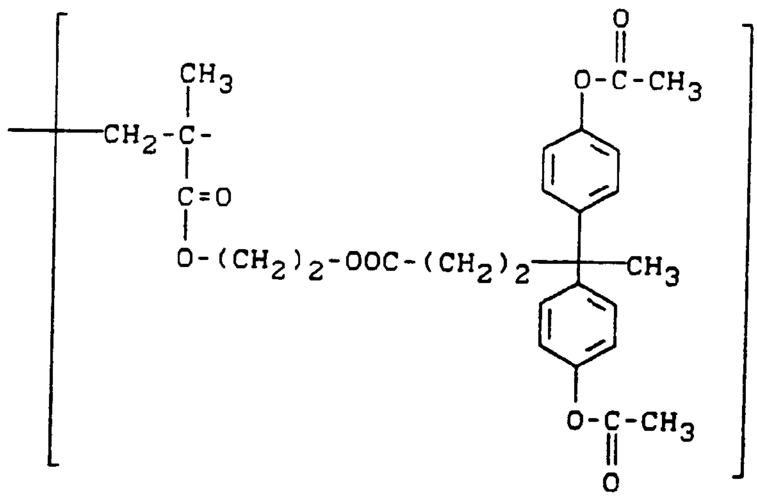

Seine Zusammensetzung ergibt sich zu

| Polymer | Gew.-% des Monomeren 1.3 im Polymerisat |
|---|---|
| 2.1 | 100 |
| 2.2 | 71 |
| 2.3 | 73 |
| 2.4 | 72 |

ermittelt durch gaschromatographische Restmonomerbestimmung und Gelpermeationschromatographie (GPC).

Die Polymerisate gemäß Beispiel 2.1 bis 2.4 besitzen folgende Eigenschaften:

| Polymerisat | $[\eta]_{DMF}$ | Aussehen bei Raumtemperatur |
|---|---|---|
| 2.1 | 0,63 | farbloses Pulver |
| 2.2 | 0,71 | farbloses Pulver |
| 2.3 | 0,73 | farbloses Pulver |
| 2.4 | 0,78 | farbloses Pulver |

Die Polymerisate sind löslich, z.B. in Dimethylformamid, Tetrahydrofuran, Aceton; die entstehenden Lösungen lassen sich zu transparenten Filmen gießen.

Die chemische Einheitlichkeit der Copolymeren gemäß Beispiel 2.2 bis 2.4 wurde durch Gelpermeationschromatographie (GPC) bewiesen.

[η] Intrinsiv-Viskosität gemessen in Dimethylformamid bei 25°C.

Beispiel 3 (erfindungsgemäß):

20 Gew.-Teile gemäß Beispiel 1.3 werden in einer Lösung von 0,4 Gew.-Teilen Natriumsalz von $C_{14}$-$C_{18}$-Alkylsulfonsäuren in 45 Gew.-Teilen Wasser emulgiert, mit 0,1 Gew.-Teilen tert.-Dodecylmercaptan versetzt und die Emulsion auf 75°C erhitzt. Nach Zugabe von 0,09 Gew.-Tl. Kaliumperoxodisulfat wird 10 Stunden bei 75°C gerührt. Es wird ein Latex mit einem Feststoffgehalt von 30 % erhalten. Die Emulsion wird mit einer wäßrigen Magnesiumsulfat-Lösung koaguliert und das abfiltrierte Polymerisat gewaschen und getrocknet. Ausbeute: 17 Gew.-Teile (85 %).

Die Intrinsic-Viskosität des Produktes beträgt [η} = 0,8 (gemessen in Dimethylformamid). Das Polymerisat ist strukturidentisch mit dem des Beispiels 2.1.

Das Polymerisat ist löslich in Tetrahydrofuran, Dimethylformamid und Aceton, dagegen unlöslich in Toluol, Methylenchlorid und Wasser.

Das Polymer läßt sich zur Herstellung transparenter Gießfolien verwenden.

Beispiel 4 (erfindungsgemäß):

50 Gew.-Teile der Emulsion gemäß Beispiel 3 (Feststoffgehalt: 30 %) werden mit 6 Gew.-Teilen konzentrierter wäßriger Ammoniak-Lösung (25 %ig) versetzt, mit 70 Gew.-Teilen Wasser verdünnt und 18 Stunden bei 70°C gerührt. Anschließend wird die Reaktionsmischung mit verdünnter wäßriger Salzsäure auf pH = 3 gestellt, der ausgefallene Feststoff abfiltriert, mit Wasser gewaschen und getrocknet. Ausbeute: 11 Gew.-Teile (90 %).

IR (KBr): $\nu$ = 3650 - 3100, 3000, 1740, 1620, 1600, 1510, 1440, 1240, 1180, 840 $cm^{-1}$.

Das Polymerisat bildet in wäßriger Natronlauge hochviskose, fließfähige Lösungen, z.B. lassen sich aus 10 gew.-%igen Lösungen feste Folien gießen.

Das heißt: das Natriumsalz des neuen Polymerisats ist gut wasserlöslich während das freie Phenol bei Raumtemperatur schwer löslich ist.

Das Polymerisat ist praktisch vollständig verseift und besteht somit vorwiegend aus der Struktureinheit

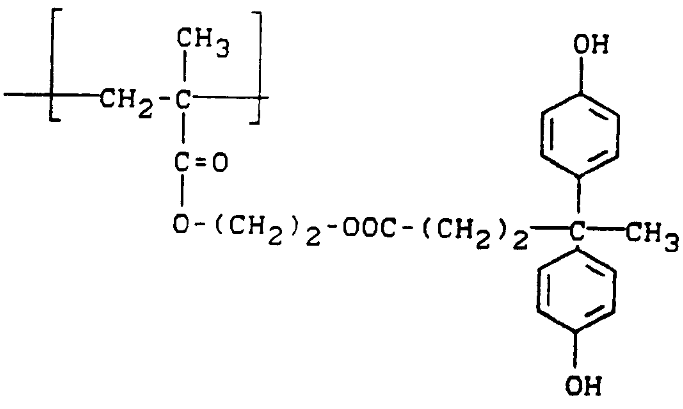

Beispiel 5

5 Gew.-Teile Polymerisat gemäß Beispiel 3 werden mit 2,5 Gew.-Teilen Bisphenol A-diglycidylether und 0,015 Gew.-Teilen Natriumhydroxid 4 Stunden auf 125°C erhitzt. Dabei erhärtet die Schmelze unter Bildung des Epoxidharzes. Das eingesetzte Polymerisat gemäß Beispiel 3 kann durch Lösungsextraktion zu über 60 % aus dem Epoxidharz abgetrennt werden.

Beispiel 6

5 Gew.-Teile Polymerisat gemäß Beispiel 4 werden mit 2,5 Gew.-Teilen Bisphenol A-diglycidylether und 0,015 Gew.-Teilen Natriumhydroxid 4 Stunden auf 125°C erhitzt. Hierbei bildet sich das Epoxidharz, aus dem das Polymerisat gemäß Beispiel 4 durch Lösungsextraktion praktisch nicht mehr abtrennbar ist; das Polymerisat gemäß Beispiel 4 hat also mit dem Epoxid unter Vernetzung reagiert.

## Patentansprüche

1. Bisphenolderivate der Formel (I)

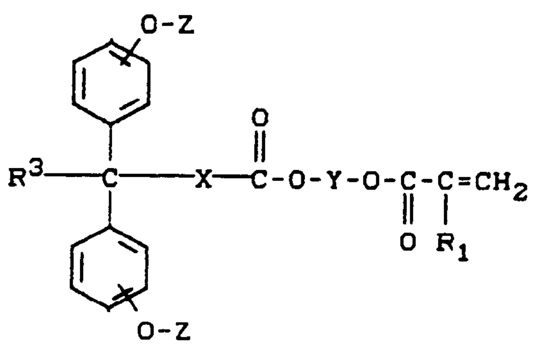

(I)

worin

| | |
|---|---|
| $R^1$ | Wasserstoff, $C_1$-$C_4$-Alkyl, |
| $R^2$ | $C_1$-$C_{12}$-Alkyl, |
| $R^3$ | $C_1$-$C_{12}$-Alkyl, |
| X | $C_1$-$C_{12}$-Alkylen, |
| Y | $C_1$-$C_{12}$-Alkylen, |
| Z | Wasserstoff, |

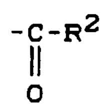

bedeuten.

2. Polymerisate enthaltend Struktureinheiten der Formel (II)

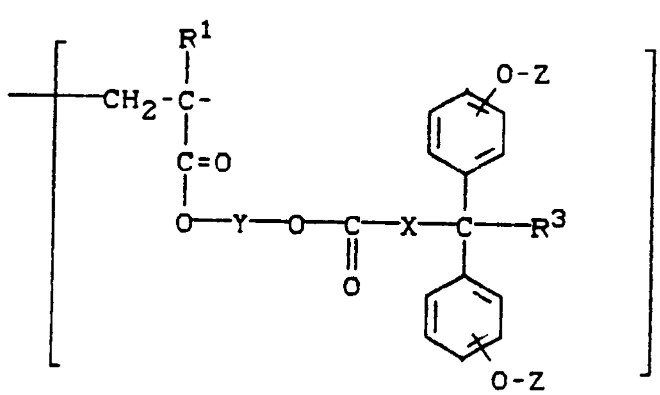

(II)

worin

$R^1$, $R^2$, $R^3$, X, Y und Z die in Anspruch 1 genannte Bedeutung haben.

## Claims

1. Bisphenol derivatives corresponding to formula (I)

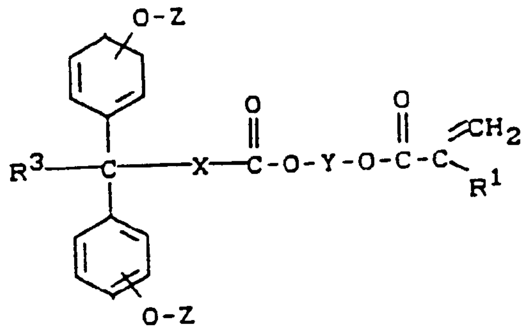

(I)

in which

| | |
|---|---|
| $R^1$ | is hydrogen, $C_{1-4}$ alkyl, |
| $R^2$ | is $C_{1-12}$ alkyl, |
| $R^3$ | is $C_{1-12}$ alkyl, |
| X | is $C_{1-12}$ alkylene, |
| Y | is $C_{1-12}$ alkylene, |
| Z | is hydrogen, |

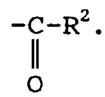

2. Polymers containing structural units corresponding to formula (II)

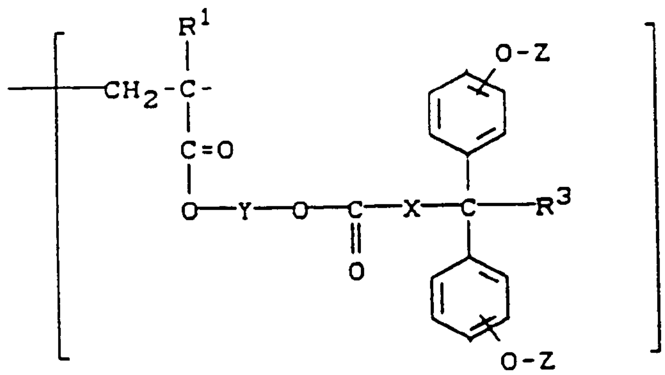

(II)

in which

$R^1$, $R^2$, $R^3$, X, Y and Z are as defined in claim 1.

## Revendications

1. Dérivés bisphénoliques de formule (I)

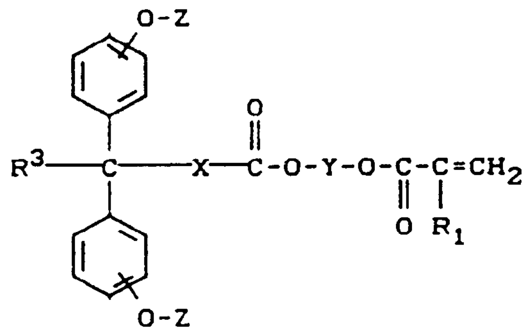

(I)

dans laquelle

$R^1$ représente l'hydrogène, un groupe alkyle en $C_1$ à $C_4$,

$R^2$ est un groupe alkyle en $C_1$ à $C_{12}$,

$R^3$ est un groupe alkyle en $C_1$ à $C_{12}$,

X est un groupe alkylène en $C_1$ à $C_{12}$,

Y est un groupe alkylène en $C_1$ à $C_{12}$,

Z est l'hydrogène, un groupe

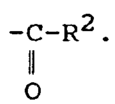

**2.** Polymères contenant des motifs structuraux de formule (II)

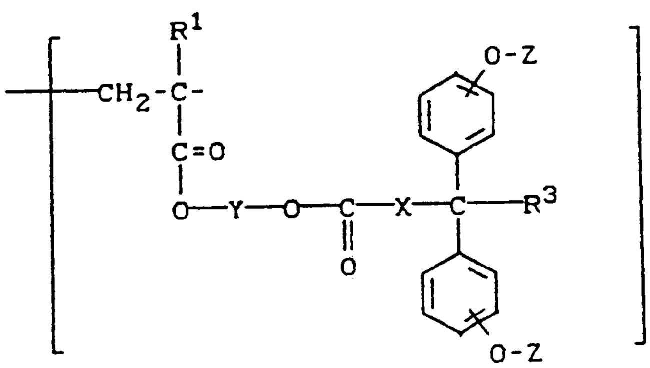

(II)

dans laquelle

$R^1$, $R^2$, $R^3$, X, Y et Z ont la définition indiquée dans la revendication 1.